**Europäisches Patentamt**

**European Patent Office**

**Office européen des brevets**

(11) Publication number : **0 564 121 A2**

# EUROPEAN PATENT APPLICATION

(21) Application number : **93302020.8**

(51) Int. Cl.⁵ : **A61K 35/74**

(22) Date of filing : **17.03.93**

(30) Priority : **29.03.92 IL 101410**

(43) Date of publication of application :
**06.10.93 Bulletin 93/40**

(84) Designated Contracting States :
**AT BE CH DE DK ES FR GB GR IE IT LI LU MC
NL PT SE**

(71) Applicant : **ERA-MASIS LTD.,**
**c/o Caspi & Co., 33 Yavetz Street**
**Tel Aviv 65 258 (IL)**

(72) Inventor : **Karapetyan, Anait, Dr. c/o Caspi &
Co.**
**33 Yavetz Street**
**Tel Aviv 65 258 (IL)**

(74) Representative : **Hardisty, David Robert et al
BOULT, WADE & TENNANT 27 Furnival Street
London EC4A IPQ (GB)**

(54) Formulation for the treatment of cancer comprising a bacterial extract.

(57) A bacterial formulation, for use in the treatment or prevention of cancer, comprises, together with at least one pharmaceutically acceptable diluent, carrier or adjuvant, an active ingredient selected from (i) and (ii), namely : (i) at least three oncolytic strains of bacteria from at least two bacterial species, wherein said strains are obtainable or derivable from human intestinal microflora ; and (ii) at least one oncolytic strain of bacteria selected from Escherichia coli A.T.C.C. 55373, Escherichia coli A.T.C.C. 55374, Escherichia coli A.T.C.C. 55375 and Streptococcus faecalis G35 A.T.C.C. 55376. The specified strains are also the subject of the invention.

EP 0 564 121 A2

Jouve, 18, rue Saint-Denis, 75001 PARIS

The present invention relates to a bacterial formulation and method for the treatment of cancer.

In general terms, the three methods in widespread clinical use for the treatment of cancer at the present time are radiation therapy, chemotherapy and surgical excision of tumors, which may be employed in combination. However, use of these methods in order to eliminate cancer cells in the body will not necessarily be complete or ultimately successful, in part because there is a failure rate associated with any therapeutic or surgical method, but more particularly because if a patient has a tendency to cancer because of a defect in the immune system, or more precisely because of an insufficiency or instability in inherent nonspecific antitumor protection in the body, cancer cells will be again generated. Thus, an agent to eliminate any defect in the body's nonspecific antitumor protection would seem to be indicated, but the present inventors could find no evidence of any such agents in the literature.

Bodily health is known to be affected by the nature of the intestinal flora, which apparently influences, for example, metabolic processes and both local and general body immune response. It has also been known for some time that certain of the intestinal flora bacteria of normal humans have oncolytic activity, and that there exists a relationship between intestinal microfloral composition and cancer morbidity, see e.g., Oleynik, S.F. and Panchishina, M.V., "About Coliflora and Cancerolycity and Carcinogenicity of the Intestine", Vrachebnoyedelo, 1968, 5: 13-17. However, mere knowledge of a relationship between the intestinal microflora, the immune system and cancer has not resulted up to now in the development of a reliable method for the treatment of cancer.

A small number of patents assert that particular bacteria can be used for the treatment of cancer, although the fact remains that these published patents (or the work on which they were based, or any similar work) have not resulted effectively in the introduction of a bacterial method of treatment into clinical practice. In US 3192116, spores of a particular strain Clostridium butyricum, isolated from humus soil and cultured, were said to show activity against various tumors. In FR 2088231, a suspension of inactivated Brucella bacteria in which 10-50% bacterial cells are whole and the remainder a lysate, was said to be useful in increasing the non-specific immunity of an organism, and for the treatment of tumors.

GB 1587244 discloses a particular strain of Streptococcus faecalis isolated from the air in Italy, which is said to be useful for combatting and diagnosing neoplasms in humans and animals. However, from animal tests which are described in GB 1587244 (no in vivo tests on humans are described), it is clear that the effectiveness of this bacterium in treating neoplasms depends on the number of days for which it is cultured and whether the culture is oxygenated or grown under hypoxic conditions. Even at the level of animal tests the results do not present a consistent picture, and it is apparent, for these reasons, why it is unlikely that the strain described in this patent would ever be used as suggested therein, for clinical treatment in practical terms.

The entire disclosures of the above-mentioned patents are explicitly incorporated herein by reference. The published literature (including patents) thus supports a relationship between bacteria, non-specific immunity and potential for the treatment of cancer, but suggests to the skilled person that a considerable research effort would be necessary in order to attain a situation where a bacterial method for the treatment of cancer would (at least) constitute an important feature of clinical practice, in addition to the presently recognized methods of radiation therapy, chemotherapy and surgery.

It is an object of the present invention to provide a bacterial formulation and method for the treatment of cancer, as well as a method for the prevention of cancer. Other objects of the invention will appear from the description which follows.

The present invention accordingly provides in one aspect a bacterial formulation, for use in the treatment or prevention of cancer, comprises, together with at least one pharmaceutically acceptable diluent, carrier or adjuvant, an active ingredient selected from (i) and (ii), namely: (i) at least three oncolytic strains of bacteria from at least two bacterial species, wherein said strains are obtainable or derivable from human intestinal microflora; and (ii) at least one oncolytic strain of bacteria selected from Escherichia coli A.T.C.C. 55373, Escherichia coli A.T.C.C. 55374, Escherichia coli A.T.C.C. 55375 and Streptococcus faecalis G35 A.T.C.C. 55376. The specified strains are also the subject of the invention.

The bacterial formulation of the invention provides a method for the treatment of cancer in a human or non-human mammal, which comprises administering to a subject diagnosed for cancer, an effective cancer treating amount of a bacterial formulation according to the invention; as well as a method for the prevention of cancer in a human or non-human mammal, which comprises administering to a subject not diagnosed for cancer, an effective cancer preventing amount of a bacterial formulation according to the invention.

In a preferred embodiment of the invention, the formulation comprises as active ingredient at least four such oncolytic strains. In another embodiment, the at least two bacterial species comprise Escherichia coli and Streptococcus faecalis, and may comprise, e.g., three strains of Escherichia coli and one strain of Streptococcus faecalis. More specifically the formulation may comprise the four specific bacterial strains mentioned above, samples of which were deposited (1) at the L.A. Tarasevitch Research Institute for Standardization and Control of Medico-Biological Preparations, Moscow, and in respect of which certificates nos. 01-07/208, /209, /210 and /211 have been issued, dated June 8, 1983, and (2) with the American Type Culture Collection (A.T.C.C.) under the Budapest Treaty, on November 13, 1992.

Escherichia coli G35 strain no. 1-59, A.T.C.C. Designation 55373.

Cultural and morphological features indicate that the strain belongs to the Escherichia genus. The straight rods of 1.1 - 1.5 x 2.0 - 6.0 mm (alive) occur separately or in pairs, and are gram-negative. They are mobile, and move using peritracheal cilia. They form pink-colored colonies in endomedium; nutritional agar colonies are smooth, moist, greyish with regular edges; gelatin colonies are turbid, greyish-white, moist. When grown on potato they give an extensive yellowish diffused coating. The strain is preserved in 2% plain agar, in a refrigerator at 4-8°C, with weekly reincubation. Reproduction is by incubation at 37°C (thermostat) for 24 hours. Oncolytic activity is shown under incubation at 37°C (thermostat) for 2-6 hours in contact with tumor cells, in vitro, as well as under in vivo conditions by administration per os.

The culture generates indole but not hydrogen sulfide, gives negative Voges-Proskauer reaction and positive reaction with methyl red. It does not eliminate urea and does not utilize citrate. It does not ferment rhamnose, raffinose, and salicin. Glucose and other carbohydrates are fermented producing pyruvate, which is then converted to lactic, acetic and formic acids. Part of the formic acid is broken down by the complex hydrogen lyase enzymatic system into equal quantities of carbon dioxide and hydrogen. The culture has weak hemolytic properties. The strain is resistant to kanamycin, oxacillin, erythromycin, methicillin, polymyxin, ampicillin, penicillin, oleandomycin, neomycin, streptomycin, rheotomycin and tetracycline, and has low sensitivity to carbenicillin, gentamycin and levomycetin.

Escherichia coli G35 strain no. 2-60, A.T.C.C. Designation 55374.

Cultural and morphological features indicate that the strain belongs to the Escherichia genus. The straight rods of 1.1 - 1.5 x 2.0 - 6.0 mm (alive) occur separately or in pairs, and are gram-negative. They are mobile, and move using peritracheal cilia. They form pink-colored colonies in endomedium; nutritional agar colonies are smooth, moist, greyish with regular edges; gelatin colonies are turbid, greyish-white, moist. When grown on potato they give an extensive yellowish diffused coating. The strain is preserved in 2% plain agar, in a refrigerator at 4-8°C, with weekly reincubation. Reproduction is by incubation at 37°C (thermostat) for 24 hours. Oncolytic activity is shown under incubation at 37°C (thermostat) for 2-6 hours in contact with tumor cells, in vitro, as well as under in vivo conditions by administration per os.

The culture generates indole but not hydrogen sulfide, gives negative Voges-Proskauer reaction and positive reaction with methyl red. It does not eliminate urea and does not utilize citrate. It does not ferment dulcite, salicin or cellobiose, but weakly ferments lactose and saccharose (by 6-10 days). Glucose and other carbohydrates are fermented producing pyruvate, which is then converted to lactic, acetic and formic acids. Part of the formic acid is broken down by the complex hydrogen lyase enzymatic system into equal quantities of carbon dioxide and hydrogen. The culture has weak hemolytic properties.

The strain is resistant to kanamycin, oxacillin, erythromycin, methicillin, polymyxin, ampicillin, penicillin, oleandomycin, neomycin, streptomycin, rheotomycin and tetracycline, and has low sensitivity to carbenicillin, gentamycin and levomycetin.

Escherichia coli G35 strain no. 3-61, A.T.C.C. Designation 55375.

Cultural and morphological features indicate that the strain belongs to the Escherichia genus. The straight rods of 1.1 - 1.5 x 2.0 - 6.0 mm (alive) occur separately or in pairs, and are gram-negative. They are mobile, and move using peritracheal cilia. They form pink-colored colonies in endomedium; nutritional agar colonies are smooth, moist, greyish with regular edges; gelatin colonies are turbid, greyish-white, moist. When grown on potato they give an extensive yellowish diffused coating. The strain is preserved in 2% plain agar, in a refrigerator at 4-8°C, with weekly reincubation. Reproduction is by incubation at 37°C (thermostat) for 24 hours. Oncolytic activity is shown under incubation at 37°C (thermostat) for 2-6 hours in contact with tumor cells, in vitro, as well as under in vivo conditions by administration per os.

The culture generates indole but not hydrogen sulfide, gives negative Voges-Proskauer reaction and positive reaction with methyl red. It does not eliminate urea and does not utilize citrate. It does not ferment rhamnose, raffinose and salicin. Glucose and other carbohydrates are fermented producing pyruvate, which is then converted to lactic, acetic and formic acids. Part of the formic acid is broken down by the complex hydrogen lyase enzymatic system into equal quantities of carbon dioxide and hydrogen. The culture has weak hemolytic properties.

The strain is resistant to kanamycin, oxacillin, erythromycin, methicillin, polymyxin, ampicillin, penicillin, oleandomycin, neomycin, streptomycin, rheotomycin and tetracycline, and has low sensitivity to carbenicillin, gentamycin and levomycetin.

Streptococcus faecalis G35 strain no. 4-62, A.T.C.C. Designation 55376.

Cultural and morphological features indicate that the strain belongs to the Streptococcus faecalis genus. The cells are spherical or oval, in pairs or diversified chains, diameter less than 2 mm. They are stable, do not form endospores and are gram-positive. The strain is preserved in 2% plain agar, in a refrigerator at 4-8°C, with weekly reincubation. Reproduction is by incubation at 37°C (thermostat) for 24 hours. Oncolytic activity is shown under incubation at 37°C (thermostat) for 2-6 hours in contact with tumor cells, in vitro, as well as under in vivo conditions by administration per os.

The culture grows in 6.5% NaCl broth at pH 9.6, or in milk with 0.1% methylene blue. For growth in plain media, folic acid is required. It ferments arabinose and sorbite, but does not produce ammonia from arginine. It contains Lancefield O and D group antigens.

The strain is resistant to kanamycin, oxacillin, erythromycin, methicillin, polymyxin, ampicillin, penicillin, oleandomycin, neomycin, streptomycin, rheotomycin and tetracycline, and has low sensitivity to carbenicillin, gentamycin and levomycetin.

In a particular embodiment of the invention, the numbers of bacteria present in the formulation may be in the ratio of 100-390 Escherichia coli to 100 Streptococcus faecalis, e.g., when three species of Escherichia coli and one of Streptococcus faecalis are present in the formulation, then the numbers of bacteria present in the formulation may be in the ratio of 35-130 (such as 35-70 or 70-130) for each strain of Escherichia coli to 100 Streptococcus faecalis.

In a presently preferred embodiment, the bacterial formulation of the invention is in unit dosage form and comprises 25-30 billion bacteria of each of the four strains (i.e. three of Escherichia and one of Streptococcus), or a sub-multiple of such formulation where the bacteria are present in the same ratio.

In an alternative presently preferred embodiment, the bacterial formulation is in unit dosage form and comprises 15-30 billion bacteria of each of three strains of Escherichia coli and 25-30 billion of one strain of Streptococcus faecalis, or a sub-multiple of such formulation where the bacteria are present in the same ratio.

Insofar as an objective of the invention is to enable the bacterial formulation to act internally in the body, the formulation may be adapted for the usual means of administration known in the art, e.g. for oral, parenteral, rectal or transdermal administration. The use of diluents, carriers and adjuvants is well known in the pharmaceutical art, and is elaborated e.g. in publications such as above mentioned GB 1587244.

The following non-limitative exemplification is intended to illustrate the present invention, including its utility.

Preparation of the Bacterial Formulation containing four strains

Fresh milk (1l) is boiled gently in a clean vessel for 10-15 minutes, covered, cooled to 36-38°C, and 30-35 g of a mixture (stored at -5 to +5°C) of equal volumes of Escherichia coli G35 strain no. 1-59 A.T.C.C. 55373, Escherichia coli G35 strain no. 2-60 A.T.C.C. 55374, Escherichia coli G35 strain no. 3-61 A.T.C.C. 55375 and Streptococcus faecalis G35 strain no. 4-62 A.T.C.C. 55376 (the concentration of each strain being 1.5-2.0 billion bacteria/ml) was added, the whole being stirred with a sterile spoon. The vessel was tightly covered, insulated and allowed to stand for 3-4 hours at external ambient temperature, when the internal temperature was about 35°C, and was then stored at -5 to +5°C. A slightly acidic taste, non-coagulation of the milk and fluidity of the mixture confirm that the formulation is ready for use. Further quantities may be prepared similarly. (Alternatively an aq. NaCl solution can be similarly prepared.) The 1l of formulation is taken orally in 100 g portions morning and evening for 5 days. An exemplary total period of administration may be 35 days, 100 g being taken morning and evening. (Formulations containing the single strains were prepared similarly.)

Utility of the Bacterial Formulation

1. The above formulation containing four bacterial strains was tested in volunteers, each of whom received a single dose of about 100 billion bacteria, per os. No pathological deviations were found. Results showing a significant subsequent increase in intestine microflora oncolytic activity are shown in Table I, below. (Oncolytic activity in Table I and TCNI in Table II mean the Tumor Cell Necrosis Index, c.f. IL 101409.)

2. 22 children who suffered from the Chernobyl event were diagnosed according to IL 101409 and were found to have a TCNI in the range 40-52%. They were subjected to clinical, endoscopic, X-ray and morphological examination, and were found to have inter alia intestine microflora disturbances; 9 of the patients had intestinal dysbacteriosis. They took the four-strain formulation of the invention for 1 month along the lines indicated at the end of the formulation example (above), the actual dosage rate being tailored to each patient's circumstances including size and weight of the patients. Results are indicated in Table II below, showing a significant increase in the TCNI value and elimination of dysbacteriosis, after taking the formulation. Additionally, all the children showed a general improvement in health and appetite, absence of discomfort, absence of negative responses and good restoration of the gastric function.

## TABLE I

| Group | Amount | Oncolytic activity | | Validity |
|---|---|---|---|---|
| | | x | y | |
| Relatively healthy | 6 | 51.5±2.7 | 61.5±2.1 | 95 |
| Cancer patients | 4 | 40.5±0.57 | 52.5±2.0 | 99.9 |

x = prior to treatment, y = following treatment

TABLE II

| No. | Patient | Age (years) | TCNI(%) x | TCNI(%) y | Intestinal dysbacteriosis x | Intestinal dysbacteriosis y |
|-----|---------|------|---|---|---|---|
| 1 | Sirenok Natasha | 8 | 40 | 48 | + | − |
| 2 | Shevchenko Tamara | 15 | 40 | 50 | + | − |
| 3 | Potapenko Sasha | 13 | 40 | 60 | | − |
| 4 | Polishchuk Lena | 8 | 42 | 48 | | − |
| 5 | Vasilenko Tanya | 14 | 42 | 48 | | − |
| 6 | Sidorenko Anya | 9 | 42 | 50 | | + |
| 7 | Matushenko Sasha | 12 | 42 | 50 | | − |
| 8 | Churikova Ira | 7 | 42 | 50 | | − |
| 9 | Churikova Sasha | 10 | 42 | 48 | | + |
| 10 | Matushenko Sergei | 10 | 44 | 56 | | − |
| 11 | Avgrimenko Tolya | 8 | 44 | 60 | | − |
| 12 | Polishchuk Denis | 15 | 44 | 50 | | + |
| 13 | Kalmaz Anton | 12 | 44 | 52 | | + |
| 14 | Sirenok Sasha | 13 | 44 | 56 | | − |
| 15 | Krutko Andrei | 9 | 46 | 50 | | + |
| 16 | Sidorenko Sveta | 11 | 46 | 80 | | + |
| 17 | Ushchapovsky Denis | 12 | 48 | 54 | | + |
| 18 | Polishchuk Sergei | 10 | 48 | 54 | | − |
| 19 | Vaks Anya | 7 | 50 | 70 | | − |
| 20 | Polishchuk Nina | 17 | 50 | 70 | | − |
| 21 | Kachur Oxana | 11 | 52 | 80 | | − |
| 22 | Kachur Sasha | 7 | 54 | 80 | | − |

x = prior to treatment

y = following treatment

3. A four-strain bacterial formulation prepared as above was fed to separate groups of rats having different types of cancer, and using different modes and/or media for administration, with the results referred to relative weight of cancer nodes in the test animals on the 10th day following administration, shown in Tables III below. There were 10 rats in each group. It may readily be seen that there is significant improvement in the treated rats as compared with the control groups.

TABLE III: ANTITUMOR EFFECT OF BACTERIAL FORMULATION

| Cancer type | Administration | Average cancer node weight (g) | Efficiency(%) |
|---|---|---|---|
| MELANOSARCOMA | Control | 32.8 | - |
| | Per os (milk) | 12.7 | 61 |
| | Per os (0.9% NaCl) | 14.9 | 55 |
| SARCOMA 45 | Control | 13.8 | - |
| | Intramuscular (0.9% HCl) | 4.3 | 69 |
| | Per os (0.9% NaCl) | 4.7 | 65 |
| WALKER ADENOCARCINOMA | Control | 27.9 | - |
| | Per os (milk) | 7.3 | 74 |
| | Per os (0.9% NaCl) | 8.5 | 70 |
| PLIES LYMPHOSARCOMA | Control | 27.1 | - |
| | Per os (milk) | 16.1 | 40 |

4. Single-strain bacterial formulations in milk, prepared as above, were administered to rats per os, as described in the preceding paragraph. The results are shown in Table IV, which compares also the results of using the four-strain formulations under similar conditions.

TABLE IV: ANTITUMOR EFFECT OF SINGLE-STRAIN AND FOUR-STRAIN FORMULATIONS

| Cancer type | Efficiency (%) in reduction of weight of tumor nodes | | | | |
|---|---|---|---|---|---|
| | Single strains (A.T.C.C. designations) | | | | Four-strain formulation |
| | 55373 | 55374 | 55375 | 55376 | |
| MELANOSARCOMA | 52% | 48% | 65% | 50% | 61% |
| SARCOMA 45 | 76% | 80% | 82% | 51% | 98% |
| WALKER ADE-NOCARCINO-MA | 68% | 60% | 72% | 70% | 74% |
| PLIES LYMPHOSARCOMA | 30% | 38% | 36% | 32% | 40% |
| SCHWEIZ LEUCOSIS | 48% | 50% | 56% | 50% | 61% |

5. Case history

A patient, 46, was hospitalized november 30, 1990 with diagnosis of ovarian cystadenoma. Histology showed right ovarian and omental metastatic tumors, granular cell cancer with thecomatosis and metastases into the omentum and the intestinal peritoneum. Disturbance of the intestine microflora (dysbacteriosis) was found, and ovarian cancer with metastases was detected. The patient took the four-strain formulation of the invention, as prepared above, for one month as indicated at the end of the preparative example. Following treatment, dysbacteriosis had been eliminated. General improvements in the patients

7

EP 0 564 121 A2

clinical state were noted, nausea, vomiting and diarrhea had ceased, and the stools had become normal. Bacterial oncolytic activity (TCNI) had changed from 36% prior to treatment, to 58% thereafter, an increase of 22% on the TCNI scale.

Although the invention is not limited by any theory, it is believed that because of deterioration in the intestinal flora associated with cancer, the bacterial formulation in accordance with an embodiment of the invention which contains a multiplicity of bacterial strains is effective because it restores normal intestinal flora and eliminates dysbacteriosis. Given that the normal intestinal flora itself contains a spectrum of bacterial strains, it is hardly to be expected that administration of a single bacterial strain would be as effective as the formulation of the invention containing a multiplicity of strains. Nevertheless, there may well be cases where the administration of one or more strains will effect an improvement in the patient's condition. However, it is believed that the present preferred formulation of a multiplicity of "strong" oncolytically active bacterial strains of intestinal origin enables the intestinal flora to remain oncolytically active for a long period of time and to promote elimination of incipient cancerous activity in the subject's organism.

While particular embodiments of the invention have been particularly shown and/or described hereinabove, it will be appreciated that the present invention is not limited thereto, since, as will be readily apparent to skilled persons, many variations and modifications can be made to the procedures described herein.

## Claims

1. A bacterial formulation, for use in the treatment of cancer, which comprises, together with at least one pharmaceutically acceptable diluent, carrier or adjuvant, active ingredient (i) or (ii) as defined below, namely, (i) at least three oncolytic strains of bacteria from at least two bacterial species, wherein said strains are obtainable or derivable from human intestinal microflora or (ii) at least one oncolytic strain of bacteria selected from Escherichia coli G35 strain no. 1-59 A.T.C.C. 55373, Escherichia coli G35 strain no. 2-60 A.T.C.C. 55374 Escherichia coli G35 strain no. 3-61 A.T.C.C. 55375 and Streptococcus faecalis G35 strain no. 4-62 A.T.C.C. 55376.

2. A bacterial formulation according to claim 1, which comprises as active ingredient four different oncolytic strains of bacteria.

3. A bacterial formulation according to claim 1, part (i), wherein said at least two bacterial species comprise Escherichia coli and Streptococcus faecalis.

4. A bacterial formulation according to claim 1, which comprises three strains of Escherichia coli and one strain of Streptococcus faecalis.

5. A bacterial formulation according to claim 4, which comprises Escherichia coli G35 strain no. 1-59 A.T.C.C. 55373, Escherichia coli G35 strain no. 2-60 A.T.C.C. 55374, Escherichia coli G35 strain no. 3-61 A.T.C.C. 55375 and Streptococcus faecalis G35 strain no. 4-62 A.T.C.C. 55376.

6. A bacterial formulation according to any of claims 3 to 5, wherein the numbers of bacteria present in the formulation are in the ratio of 100-390 Escherichia coli to 100 Streptococcus faecalis.

7. A bacterial formulation according to claim 4, wherein the numbers of bacteria present in the formulation are in the ratio of 35-130 for each strain of Escherichia coli to 100 Streptococcus faecalis.

8. A bacterial formulation according to claim 7, wherein the numbers of bacteria present in the formulation are in the ratio of 70-130 for each strain of Escherichia coli to 100 Streptococcus faecalis.

9. A bacterial formulation according to claim 5, which is in unit dosage form and comprises 25-30 billion bacteria of each of the four strains, or a sub-multiple of such formulation where the bacteria are present in the same ratio.

10. A bacterial formulation according to claim 8, which is in unit dosage form and comprises 25-30 billion bacteria of each of the four strains, or a sub-multiple of such formulation where the bacteria are present in the same ratio.

11. A bacterial formulation according to claim 7, wherein the numbers of bacteria present in the formulation are in the ratio of 35-70 for each strain of Escherichia coli to 100 Streptococcus faecalis.

12. A bacterial formulation according to claim 5, which is in unit dosage form and comprises 15-30 billion bacteria of each of the three strains of Escherichia coli and 25-30 billion of the one strain of Streptococcus faecalis, or a sub-multiple of such formulation where the bacteria are present in the same ratio.

13. A bacterial formulation according to claim 8, which is in unit dosage form and comprises 15-30 billion bacteria of each of the three strains of Escherichia coli and 25-30 billion of the one strain of Streptococcus faecalis, or a sub-multiple of such formulation where the bacteria are present in the same ratio.

14. A bacterial formulation according to any of the preceding claims, which is adapted for oral, parenteral, rectal or transdermal administration.

15. A bacterial strain which is Escherichia coli G35 strain no. 1-59 A.T.C.C. 55373, Escherichia coli G35 strain no. 2-60 A.T.C.C. 55374, Escherichia coli G35 strain no. 3-61 A.T.C.C. 55375 or Streptococcus faecalis G35 strain no. 4-62 A.T.C.C. 55376.

16. Use of an active ingredient as defined in claim 1 for the manufacture of a medicament for the treatment of cancer in a human or non-human mammal.

17. Use of an active ingredient as defined in claim 1 for the manufacture of a medicament for the prevention of cancer in a human or non-human mammal.